# EUROPEAN PATENT SPECIFICATION

(11) **EP 0 369 749 B1**
(45) Date of publication and mention of the grant of the patent: **27.09.1995**
(21) Application number: 89311789.5
(22) Date of filing: 14.11.1989
(51) Int. Cl.: C07D 471/18, C07D 498/22, A61K 31/495

(54) **Anti-Tumor Compounds**
Antitumor-Verbindungen
Composés antitumoraux

(30) Priority: 16.11.1988 JP 289565/88
(43) Date of publication of application: 23.05.1990
(73) Proprietor: Kyowa Hakko Kogyo Co., Ltd., Chiyoda-ku, Tokyo 100 (JP)
(72) Inventor: Saito, Hiromitsu Patent Department, Chiyoda-ku Tokyo (JP); Sato, Akira Patent Department, Chiyoda-ku Tokyo (JP); Kasai, Masaji Patent Department, Chiyoda-ku Tokyo (JP); Morimoto, Makoto c/o Patent Department, Chiyoda-ku Tokyo (JP); Ashizawa, Tadashi c/o Patent Department, Chiyoda-ku Tokyo (JP)
(74) Representative: Harding, Charles Thomas

(56) References cited:
- EP-A- 0 262 984

## Description

The present invention relates to anti-tumour compounds which are derivatives of DC-52.

DC-52 is a compound represented by the formula: 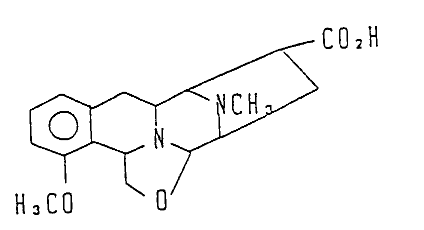
and exhibits an anti-tumor activity against lymphocytic leukemia P-388, etc., in addition to an anti-bacterial activity against various bacteria.

The preparation and properties of DC-52 are described in published unexamined Japanese patent application JP-A-170 89/82.

A first derivative of DC-52, DX-52-1, likewise having anti-tumor activity and represented by the formula: 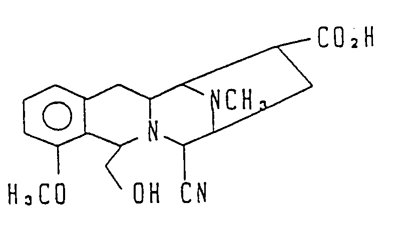
is disclosed in US Patent Specification US-A-4,650,869.

Other derivatives of DC-52 are disclosed in EP-A-0 283 521, viz. compounds of the formula: 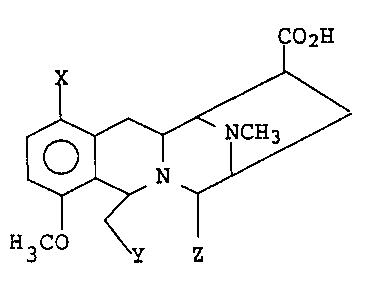
wherein X represents chlorine, bromine, iodine, hydroxyl, formyl, hydroxyiminomethyl, cyano, nitro, amino or lower alkanoylamino, Y represents hydroxyl and Z represents cyano, or Y and Z are combined together to represent -O- as -Y-Z-.

Yet further derivatives are disclosed in published unexamined Japanese patent application JP-A-183583/88, viz. compounds of the formula: 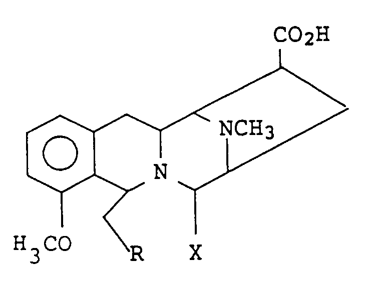
wherein R represents hydrogen, alkanoyloxy having 1 to 18 carbon atoms, oleoyloxy, linoloyloxy, linolenoyloxy, retinoyloxy, unsubstituted or substituted benzoyloxy in which the substituent is halogen or lower alkoxy, mercapto, alkanoylthio having 1 to 18 carbon atoms, oleoylthio, linoloylthio, linolenoylthio, retinoylthio, unsubstituted or substituted benzoylthio in which the substitutent is halogen or lower alkoxy, azido, amino, -NHZ [in which Z is an alpha-amino acid residue (wherein the OH of carboxyl of the alpha-amino acid is removed and the amino and/or carboxyl when it is present may be protected by their protective groups conventionally used in the peptide synthetic chemistry), pyruvoyl, citroyl or acetyl], cyano or halogen, and X represents cyano or hydroxyl.

Finally, EP-A-0 262 984 discloses further derivatives of the formula: 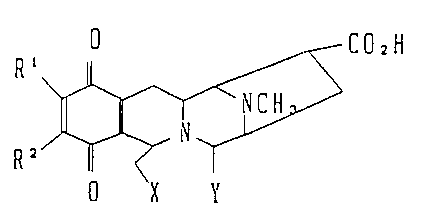
wherein each of R¹ and R² independently represents hydrogen, lower alkoxy, azido, amino, lower alkylamino, dilower alkylamino, cyclic amino, lower alkylthio, or unsubstituted or substituted arylthio, X represents hydroxyl and Y represents cyano, or X and Y are combined together to represent -O- as -X-Y-.

In accordance with the present invention, further DC-52 derivatives have been discovered having superior properties over the prior art compounds. These are compounds of the formula (I): 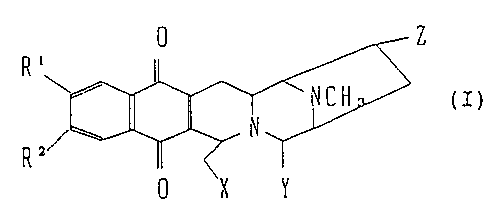
where R¹ and R² each represent hydrogen or hydroxyl, X represents hydroxyl and Y represents cyano, or X and Y are joined together and represent -O-, and Z represents carboxyl or hydroxymethyl.

Also disclosed herein are the pharmacologically acceptable salts of compounds I including the pharmacologically acceptable acid addition salts, alkali metal salts, alkaline earth metal salts and ammonium salts and pharmacologically acceptable organic base addition salts. Pharmacologically acceptable acid addition salts include pharmacologically acceptable inorganic acid addition salts such as hydrochloride, hydrobromide, hydroiodide, sulfate, phosphate and nitrate; and pharmacologically acceptable organic acid addition salts such as acetate, benzoate, maleate, fumarate, succinate, tartrate, citrate, oxalate, glyoxylate, aspartate, methanesulfonate, ethanesulfonate, propanesulfonate, methanedisulfonate, alpha-, beta-ethanedisulfonate and benzenesulfonate. Alkali metal salts include sodium salts, potassium salts, etc. and alkaline earth metal salts include calcium salt, magnesium salt, etc. Pharmacologically acceptable organic base addition salts include addition salts of ethanolamine, triethylamine, morpholine, piperidine, etc.

Also included within the scope of the invention are pharmaceutical preparations comprising the Compounds I or their pharmaceutically acceptable salts in admixture with a pharmaceutically acceptable carrier or diluent.

The compounds I can be prepared by the following steps:

### Step 1

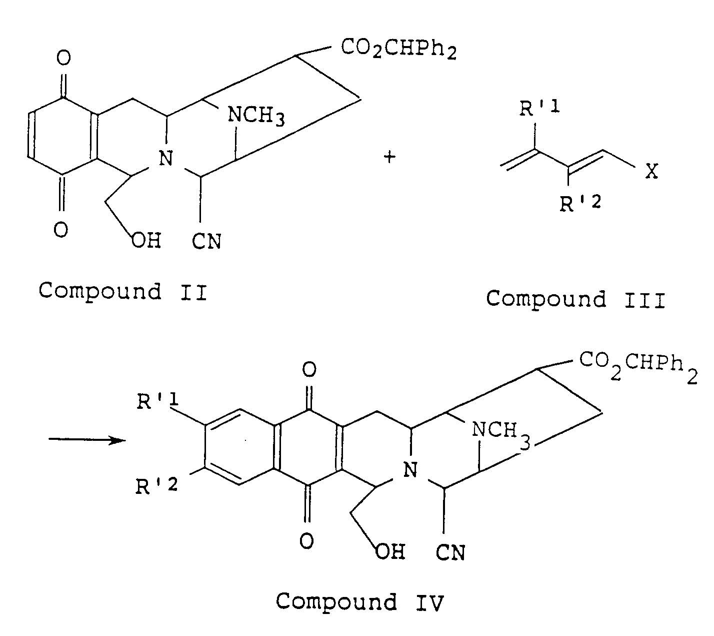

wherein Ph represents phenyl, each of R'¹ and R'² independently represents hydrogen or trimethylsiloxy, and X represents acetoxy, methoxy or trimethylsiloxy.

The starting compounds II are known from EP-A-0 262 984, and compounds III are either commercially available or can be readily synthesized by known processes. In Step 1 compound IV is obtained by reacting compound II with compound III in an inert solvent such as benzene, toluene, xylene, etc. Compound III is used in an amount of from 1 to 3 molar equivalents based on compound II. The reaction is carried out at 50 to 130°C and is usually complete within from 5 to 46 hours.

### Step 2

In the case of compounds IV, where R'¹ and R'² both represent trimethylsiloxy; or one of R'¹ and R'² represents trimethylsiloxy and the other represents hydrogen, these can be reacted with a desilylating agent in an inert solvent such as methanol, ethanol, tetrahydrofuran, dioxane, acetonitrile, water, etc. to produce the compound V: 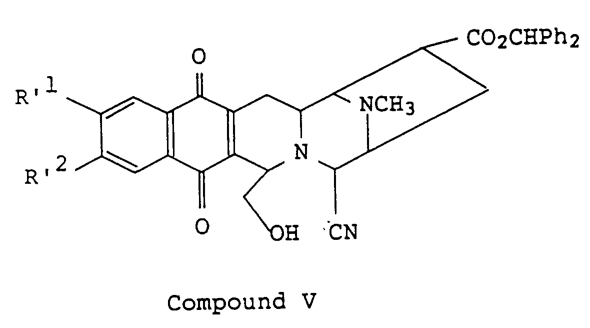
where R'¹ and R'² are either both hydroxyl or one is hydroxyl and the other hydrogen depending on the values of R'¹ and R'² in compound IV.

Suitable desilylating agents are potassium carbonate, tetra-n-butylammonium fluoride, etc. and are used in an amount of from 1 to 10 molar equivalents based on compound IV. The reaction is carried out at 0 to 60°C and is usually complete within from 1 to 15 hours.

### Step 3

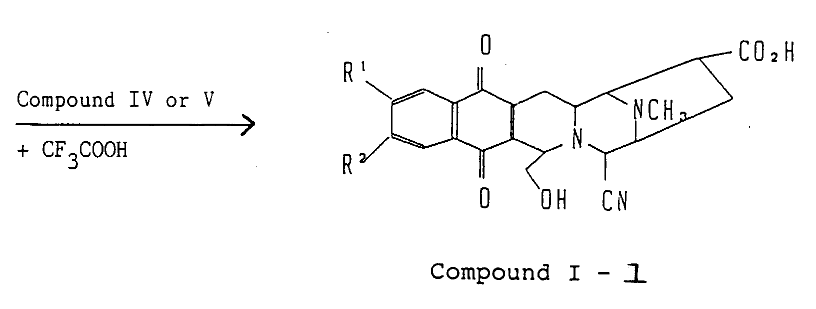

From the compounds V, or the compounds IV wherein R'¹ and R'² are both hydrogen, the compounds I-1 can be obtained by reacting with trifluoroacetic acid in an inert solvent such as dichloroethane or chloroform, etc. The trifluoroacetic acid is preferably used in an amount of at least 5 molar equivalents upto a large excess based on a compound V (or IV as the case may be). The reaction is carried out at 0 to 50°C and is usually complete within 1 to 10 hours.

### Step 4 (Alternative)

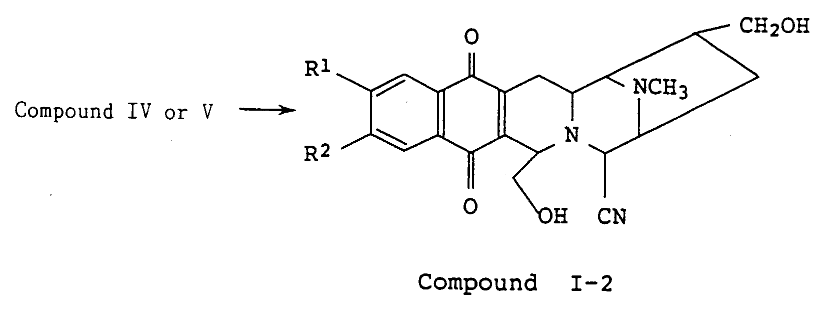

Alternatively, the compounds V or the compounds IV where R'¹ and R'² are both hydrogen can be reduced by reaction with a reducing agent in an inert solvent such as tetrahydrofuran, diethyl ether, toluene, benzene, methanol or ethanol, etc. to give the compounds I-2. Suitable reducing agents are LiAlH₄, LiBH₄, NaBH₄, etc. and are usually used in an amount of from 3 to 10 molar equivalents based on the compound IV or V. The reaction is carried out at -10 to 70°C and is usually complete within 1 to 10 hours.

### Step 5 (Alternative)

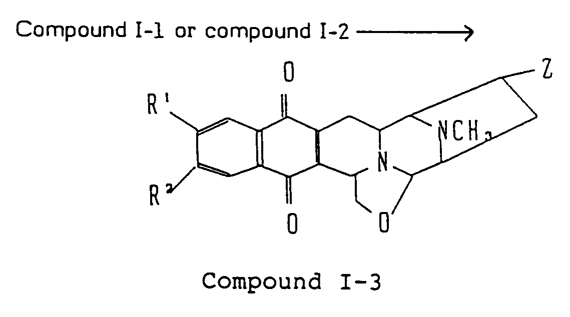

The compounds I-3 can be obtained by reacting a compound I-1 or compound I-2 with a silver salt in an inert solvent. Suitable inert solvents are acetonitrile, methanol, ethanol, tetrahydrofuran, dioxane, etc. Suitable silver salts are silver nitrate, silver chlorate, silver perchlorate, silver fluoride, etc. Usually the silver salt is used in an amount of from 1 to 5 molar equivalents based on the compound I-1 or compound I-2. The reaction is usually carried out at 0 to 50°C and is usually complete within 30 minutes to 5 hours.

After completion of the reaction of each step, the reaction can be stopped by adding an acid, water, a buffer or the like, if necessary, and the mixture is concentrated as such, or after extraction with a nonaqueous solvent such as ethyl acetate, chloroform, etc. Alternatively, the reaction mixture is concentrated as such and purified. The purification can be carried out by column chromatography, thin layer chromatography, preparative high performance liquid chromatography, recrystallization or the like.

Compounds I and the pharmacologically acceptable salts thereof have a strong anti-tumor activity against lymphocytic leukemia P-388, etc.

Structures of typical compounds I are listed in Table 1.

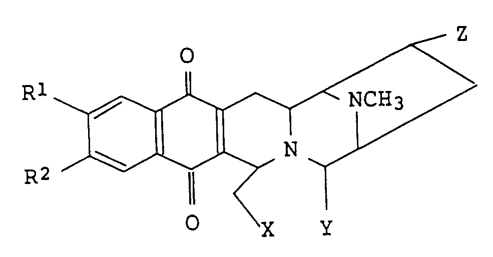

**Table 1**

| Compound No. | R¹ | R² | X | Y | Z |
|---|---|---|---|---|---|
| 1 | H | H | OH | CN | CO₂Na |
| 2 | H | H | -O - | | CO₂H |
| 3 | H | H | OH | CN | CH₂OH |
| 4 | H | H | -O - | | CH₂OH |
| 5 | OH | H | OH | CN | CO₂H |
| | or | | | | |
| | H | OH | | | |
| 6 | OH | H | -O - | | CO₂H |
| | or | | | | |
| | H | OH | | | |

The anti-tumor activity of the compounds I is demonstrated below by an experimental example.

### Experimental example

Lymphocytic leukemia P-388 tumor cells (1 x 10⁶) were intraperitoneally implanted into CDF male mice weighing about 22g. 24 hours after the implantation, one group consisting of 5 animals was intraperitoneally administered once with 0.2 mℓ of a solution of each drug in phosphate-buffered physiological saline. The life prolonging effect of the drugs was shown in T/C. The results are shown in Table 2.

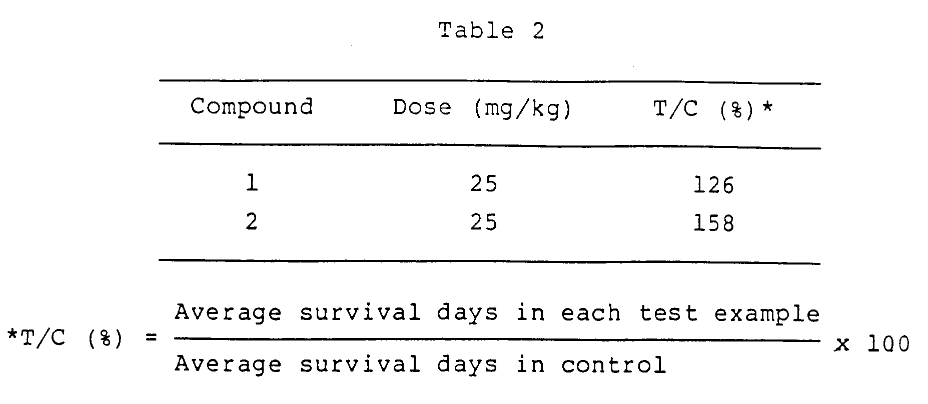

Examples and reference examples are illustrated below.

Physicochemical data exhibited in the following examples and reference examples were measured by the following instruments:
- NMR: Bruker AM400 (400 MHz)
- MS: Hitachi B-80

### Example 1 Synthesis of compound 1

In this example, 1.89g of compound a obtained in Reference example 1 was dissolved in 50 mℓ of methylene chloride. Then, 3.5 mℓ of anisole and 6 mℓ of trifluoroacetic acid (TFA) were added thereto, and the mixture was stirred at room temperature for one hour and 30 minutes. After the reaction solution was concentrated, the concentrate was dissolved in a sodium bicarbonate solution and purified by column chromatography (Diaion HP-20 180 mℓ, eluting solvent; water : methanol = 1 : 0 to 4 : 1 v/v) to obtain 1.12g (79.2%) of compound 1.
¹H-NMR (DMSO-d₆ - CD₃OD) ppm; 8.11 - 8.14 (m, 2H), 7.87 - 7.92 (m, 2H), 4.66 (d, 1H, J=2.5Hz), 4.09 (m, 1H), 4.02 (m, 1H), 3.96 (bs, 1H), 3.90 (dd, 1H, J=11.4, 2.3Hz), 3.68 (dd, 1H, J=11.4, 4.1Hz), 3.51 (dd, 1H, J=9.9, 5.7Hz), 3.07 (bd, 1H, J=11.2Hz), 3.02 (dd, 1H, J=17.3, 2.5Hz), 2.70 (dt, 1H J=13.4, 6.3Hz), 2.62 (s, 3H), 2.36 (ddd, 1H, J=17.2, 10.8, 2.4Hz), 2.28 (dd, 1H, J=13.7, 10.0Hz)
SIMS (m/z); 410 (M+3)⁺ (carboxylic acid-free)

### Example 2 Synthesis of compound 2

In this example, 900 mg of compound 1 was dissolved in a mixture of 15 mℓ of acetonitrile and 15 mℓ of methanol. Then, 1.07g of silver nitrate was added thereto, and the mixture was stirred at room temperature for 2 hours and 30 minutes. Then, 1N hydrochloric acid was added thereto. After removal of the formed precipitate by filtration acetonitrile and methanol were distilled away. The resulting concentrate was purified by column chromatography (Diaion HP-20 100 mℓ, eluting solvent; water : methanol = 1 : 0 to 3 : 2 v/v) to obtain 719 mg (90.2%) of compound 2.
¹H-NMR (D₂O) ppm; 7.98 (m, 2H), 7.79 (m, 2H), 5.00 (d, 1H, J=3.3Hz), 4.34 (m, 1H), 4.29 (s, 1H), 4.05 (m, 1H), 3.87 (dd, 1H, J=12.0, 2.7Hz), 3.69 (dd, 1H, J=12.0, 3.3Hz), 3.48 (bd, 1H, J=10.7Hz), 3.42 (dd, 1H, J=10.6, 5.4Hz), 2.95 (m, 1H), 2.84 (s, 3H), 2.58 (m, 1H), 2.41 (dd, 1H, J=14.3, 10.6Hz), 2.29 (m, 1H)
SIMS (m/z); 383 (M+3)⁺

### Example 3 Synthesis of compound 3

In this example, 40 mg of LiAℓH₄ was suspended in 3 mℓ of tetrahydrofuran (THF) in an argon gas atmosphere. Under cooling to 0°C was dropwise added a solution of 180 mg of compound a obtained in Reference example 1 in 4 mℓ of THF. After stirring at 0°C for 50 minutes, 10 mg of LiAℓH₄ was added, followed by stirring for further 2 hours and 15 minutes. After stirring for 30 minutes after addition of ethyl acetate and an acetate buffer (pH 4.0), a NaHCO₃ solution was added, and the mixture was extracted three times with ethyl acetate. The extract was washed with saturated aqueous sodium chloride, dried over anhydrous sodium sulfate, and evaporated to remove the solvent. The residue was purified by column chromatography (Wako gel C-200 20 mℓ, eluting solvent; n-hexane : ethyl acetate = 1 : 1 to 1 : 2 v/v) to obtain 39.9 mg (32.3%) of compound 3.
¹H-NMR (CDCℓ₃-CD₃OD) ppm; 8.08 - 8.10 (m, 2H), 7.74 - 7.76 (m, 2H), 4.18 (m, 1H), 4.13 (bs, 1H), 3.87 (dd, 1H, J=11.6, 2.8Hz), 3.70 (dd, 1H, J=11.6, 3.8Hz), 3.62 - 3.66 (m, 2H), 3.15 (br, 1H), 3.03 (m, 1H), 2.89 (dd, 1H, J=17.7, 2.2Hz), 2.64 (bs, 3H), 2.56 (m, 1H), 2.24 (ddd, 1H, J=17.7, 11.0, 2.6Hz), 1.91 - 2.03 (m, 2H)
SIMS (m/z); 396 (M+3)⁺

### Example 4 Synthesis of compound 4

In this example, 145 mg of compound 3 was dissolved in 3 mℓ of acetonitrile and 1 mℓ of methanol. Then, 157 mg of silver nitrate was added thereto. After stirring at room temperature for 4 hours and 50 minutes, the insoluble matter was removed by filtration and the filtrate was concentrated. The concentrate was purified by column chromatography (Diaion HP-20 15 mℓ, eluting solvent, water methanol = 1 : 0 to 4 : 1 v/v) to obtain 90.2 mg (66.8%) of compound 4.
¹H-NMR (CD₃OD) ppm; 8.07 - 8.09 (m, 2H), 7.78 - 7.81 (m, 2H), 4.61 (d, 1H, J=3.1Hz), 4.41 (m, 1H), 4.20 (m, 1H), 3.89 (dd, 1H, J=11.6, 2.7Hz), 3.87 (bs, 1H), 3.73 (dd, 1H, J=11.2, 6.0Hz), 3.61 - 3.68 (m, 2H), 3.40 (m, 1H), 2.97 (s, 3H), 2.94 (dd, 1H, J=17.3, 2.5Hz), 2.32 (dd, 1H, J=14.1, 9.9Hz), 2.28 (ddd, 1H, J=17.2, 11.2, 2.3Hz), 2.08 (m, 1H)
SIMS (m/z); 369 (M+3)⁺

### Example 5 Synthesis of compound 5

In this example, 335 mg of compound b obtained in Reference example 2 was dissolved in 7 mℓ of methylene chloride. Then, 0.5 mℓ of anisole and 1 mℓ of TFA were added thereto. The mixture was stirred at room temperature for 2 hours. The reaction solution was concentrated and the residue was purified by column chromatography (Diaion HP-20 40 mℓ, eluting solvent; water methanol = 1 : 0 to 1 : 1 v/v) to obtain 234 mg (97.3%) of compound 5.
¹H-NMR (DMSO-d₆) ppm; 12.34 (br, 1H), 10.92 (s, 1H), 7.89 (d, 1H, J=8.5Hz), 7.31 (d, 1H, J=2.6Hz), 7.15 (dd, 1H, J=8.5, 2.6Hz), 4.63 (br, 1H), 4.32 (bs, 1H), 3.82 (bs, 1H), 3.71 (dd, 1H, J=11.2, 1.9Hz), 3.43 - 3.48 (m, 2H), 3.15 (m, 1H), 2.67 - 2.75 (m, 2H), 2.40 (m, 1H), 2.22 (s, 3H), 2.12 (ddd, 1H, J=16.7, 10.5, 2.2 Hz), 1.91 (m, 1H)
SIMS (m/z); 426 (M+3)⁺, 425 (M+2)⁺, 424 (M+1)⁺

### Example 6 Synthesis of compound 6

In this example, 209 mg of compound 5 was dissolved in a mixture of 3 mℓ of acetonitrile and 6 mℓ of methanol. Then, 250 mg of silver nitrate was added thereto. After stirring at room temperature for 3 hours, water was added, the insoluble matter was removed by filtration, and acetonitrile and methanol were distilled away from the filtrate. The residue was purified by column chromatography (Diaion HP-20 30 mℓ, eluting solvent; water : methanol = 1 : 0 to 1 : 1 v/v) to obtain 185 mg (94.6%) of compound 6.
¹H-NMR (DMSO-d₆) ppm; 7.83 (d, 1H, J=8.5Hz), 7.29 (d, 1H, J=2.5Hz), 7.13 (dd, 1H, J=8.5, 2.5Hz), 4.38 (t, 1H, J=6.9Hz), 3.95 (d, 1H, J=2.5Hz), 3.57 (m, 1H), 3.41 (s, 1H), 3.26 - 3.40 (2H, overlapping with absorption of water), 3.06 (m, 1H), 2.83 (dd, 1H, J=9.7, 5.4Hz), 2.72 (ddd, 1H, J=20.3, 5.8, 3.2Hz), 2.39 (ddd, 1H, J=20.2, 8.2, 3.3Hz), 2.30 (s, 3H), 2.17 (dt, 1H, J=12.5, 6.1Hz), 1.78 (dd, 1H, J=12.7, 9.8Hz)
SIMS (m/z); 399 (M+3)⁺
Structures of compounds obtained by the following reference examples and compound numbers are indicated below:

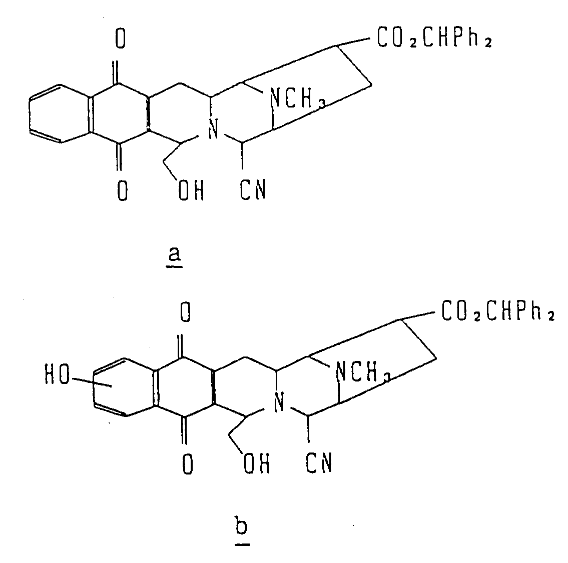

### Reference example 1 Synthesis of compound a

In this reference example, 1.0g of compound II was dissolved in 25 mℓ of benzene. Then, 0.27 mℓ of 1-acetoxy-1,3-butadiene was added thereto. The mixture was sealed in a pressure vessel and stirred at 90°C for 19 hours. The reaction solution was concentrated and purified by column chromatography (Wako gel C-200 100 mℓ, eluting solvent; chloroform) to obtain 849 mg (77.5%) of compound a.
¹H-NMR (CDCℓ₃) ppm; 8.08 - 8.11 (m, 2H), 7.72 - 7.76 (m, 2H), 7.30 - 7.40 (m, 10H), 6.92 (s, 1H), 4.16 (m, 1H), 4.04 (d, 1H, J=2.9Hz), 3.88 (dd, 1H, J=11.7, 3.2Hz), 3.76 (dd, 1H, J=11.8, 2.4Hz), 3.57.(bs, 1H), 3.52 (m, 1H), 3.16 (dd, 1H, J=9.6, 5.7Hz), 3.05 (m, 1H), 3.01 (m, 1H), 2.71 (m, 1H), 2.28 (ddd, 1H, J=18.9, 12.0, 2.7Hz), 2.18 (s, 3H), 1.98 (dd, 1H, J=13.6, 9.6Hz)
SIMS (m/z); 573(M⁺), 546, 542, 501, 374, 167

### Reference example 2 Synthesis of compound b

In this reference example, 500 mg of compound II was dissolved in 15 mℓ of benzene. Then, 0.22 mℓ of 1-methoxy-3-trimethylsiloxy-1,3-butadiene was added thereto. The mixture was sealed in a pressure vessel and stirred at 90°C for 25 hours. After distilling away the benzene, the residue was dissolved in 20 mℓ of methanol, 7.5 mg of potassium carbonate was added, and the mixture was stirred at room temperature for 3 hours and 30 minutes. After distilling away methanol, the residue was distributed between water and ethyl acetate. The ethyl acetate layer was separated, washed with saturated aqueous sodium chloride, dried over anhydrous sodium sulfate and evaporated to remove the solvent. The residue was purified by column chromatography (Wako gel C-200 80 mℓ, eluting solvent; chloroform : methanol = 1 : 0 to 100 : 1 v/v) to obtain 358 mg (62.0%) of compound b.
¹H-NMR (CDCl₃) ppm; 7.75 (br, 1H), 7.23 - 7.40 (m, 12H), 6.91 (s, 1H), 4.06 (br, 1H), 4.01 (bs, 1H), 3.73 - 3.82 (m, 2H), 3.59 (s, 1H), 3.48 (m, 1H), 3.22 (m, 1H), 2.98 (m, 1H), 2.87 (m, 1H), 2.68 (m, 1H), 2.16 (s, 3H), 2.12 - 2.24 (m, 1H), 2.00 (m, 1H)
SIMS (m/z): 592 (M+3)⁺, 565

## Claims

1. DC-52 derivatives of the formula (I): 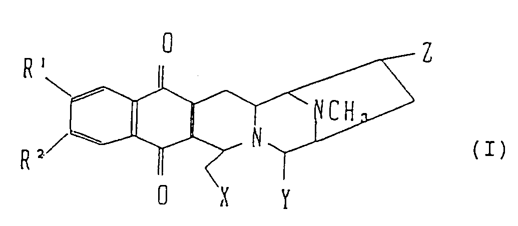 where R¹ and R² each represent hydrogen or hydroxyl, X represents hydroxyl and Y represents cyano, or X and Y are joined together and represent -O-, and Z represents carboxyl or hydroxymethyl, and their pharmacologically acceptable salts.

2. The compounds identified below as compound numbers 1 to 6, and being respectively compounds of the formula set out in claim 1 where R¹, R², X, Y, and Z have the following values:
| Compound No. | R¹ | R² | X | Y | Z |
|---|---|---|---|---|---|
| 1 | H | H | OH | CN | CO₂Na |
| 2 | H | H | -O - | | CO₂H |
| 3 | H | H | OH | CN | CH₂OH |
| 4 | H | H | -O - | | CH₂OH |
| 5 | OH | H | OH | CN | CO₂H |
| | or | | | | |
| | H | OH | | | |
| 6 | OH | H | -O - | | CO₂H |
| | or | | | | |
| | H | OH | | | |

3. Pharmaceutical preparations comprising a compound or salt as claimed in claim 1, or any one of the compounds claimed in claim 2, in admixture with a pharmaceutically acceptable carrier or diluent.

## Patentansprüche

1. DC-52 Derivate der Formel (I): 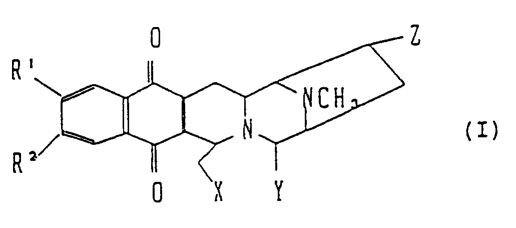 in der R¹ und R² jeweils ein Wasserstoffatom oder eine Hydroxylgruppe darstellen, X eine Hydroxylgruppe darstellt und Y eine Cyanogruppe darstellt, oder X und Y zusammen -O- darstellen, und Z eine Carboxyl- oder Hydroxymethylgruppe darstellt, und ihre pharmakologisch verträglichen Salze.

2. Nachstehend als Verbindungen Nr. 1 bis 6 identifizierte Verbindungen, wobei es sich jeweils um Verbindungen der in Anspruch 1 definierten Formel handelt, in denen R¹, R², X, Y und Z folgende Werte haben:
| Verbindung Nr. | R¹ | R² | X | Y | Z |
|---|---|---|---|---|---|
| 1 | H | H | OH | CN | CO₂Na |
| 2 | H | H | -O - | | CO₂H |
| 3 | H | H | OH | CN | CH₂OH |
| 4 | H | H | -O - | | CH₂OH |
| 5 | OH | H | OH | CN | CO₂H |
| | oder | | | | |
| | H | OH | | | |
| 6 | OH | H | -O - | | CO₂H |
| | oder | | | | |
| | H | OH | | | |

3. Arzneimittel, umfassend eine Verbindung oder ein Salz nach Anspruch 1 oder eine der Verbindungen nach Anspruch 2 in einem Gemisch mit einem pharmazeutisch verträglichen Träger oder Verdünnungsmittel.

## Revendications

1. Dérivés de DC-52, de formule (I) : 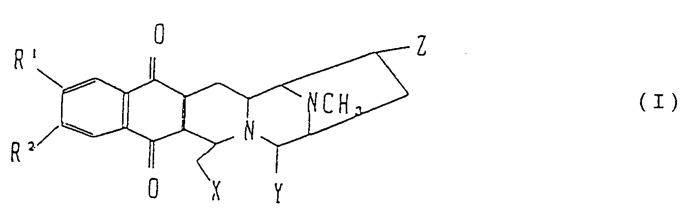 dans laquelle R¹ et R² représentent chacun un atome d'hydrogène ou un groupe hydroxyle, X représente un groupe hydroxyle et Y représente un groupe cyano ou bien X et Y sont réunis et représentent -O-, et Z représente un groupe carboxyle ou hydroxyméthyle,
et sels de ces composés, acceptables en pharmacie.

2. Composés identifiés ci-dessous comme étant les composés N° 1 à 6, qui sont respectivement des composés correspondant à la formule indiquée dans la revendication 1, dans laquelle R¹, R², X, Y et Z ont les significations suivantes :
| Composé N° | R¹ | R² | X | Y | Z |
|---|---|---|---|---|---|
| 1 | H | H | OH | CN | CO₂Na |
| 2 | H | H | - O - | | CO₂H |
| 3 | H | H | OH | CN | CH₂OH |
| 4 | H | H | - O - | | CH₂OH |
| 5 | OH | H | OH | CN | CO₂H |
| | ou | | | | |
| | H | OH | | | |
| 6 | OH | H | - O - | | CO₂H |
| | ou | | | | |
| | H | OH | | | |

3. Préparation pharmaceutique comprenant un composé ou un sel conforme à la revendication 1, ou n'importe lequel des composés conformes à la revendication 2, mélangé avec un diluant ou véhicule acceptable en pharmacie.
